(19) Europäisches Patentamt
European Patent Office
Office européen des brevets

(11) Numéro de publication : **0 017 550**
**B1**

(12) # FASCICULE DE BREVET EUROPÉEN

(45) Date de publication du fascicule du brevet : **15.12.82**

(51) Int. Cl.³ : **G 01 N 33/52**, C 12 Q 1/00

(21) Numéro de dépôt : **80400383.8**

(22) Date de dépôt : **21.03.80**

(54) **Nouveau procédé de dosage des aminoglycosides et réactifs pour la mise en oeuvre de ce procédé.**

(30) Priorité : **27.03.79 FR 7907594**

(43) Date de publication de la demande : **15.10.80 (Bulletin 80/21)**

(45) Mention de la délivrance du brevet : **15.12.82 Bulletin 82/50**

(84) Etats contractants désignés : **BE CH DE GB IT NL**

(56) Documents cités :
**BE A 872 177**
**FR A 2 348 492**
**CHEMICAL ABSTRACTS, vol. 80, no. 5, 4 février 1974, réf. no. 22451b, page 5 COLUMBUS OHIO (US).**
**CHEMICAL ABSTRACTS, vol. 86, no. 9, 28 février 1977, réf. no. 50415k, page 4 COLUMBUS OHIO (US).**
**CHEMICAL ABSTRACTS, vol. 89, no. 1, 3 juillet 1978, réf. no. 2304p, page 219 COLUMBUS ; OHIO (US).**
**CHEMICAL ABSTRACTS, vol. 89, no. 15, 9 octobre 1978, réf. no. 125287t, page 244 COLUMBUS OHIO (US).**
**INFECTION, vol. 5, no. 2, 1977 F.H. KAYSER : « Enzymatische Wertbestimmung von Aminoglykosid-Antibiotika », pages 123-125.**

(73) Titulaire : **ANVAR Agence Nationale de Valorisation de la Recherche**
**13, rue Madeleine Michelis**
**F-92522 Neuilly-sur-Seine (FR)**

(72) Inventeur : **Le Goffic, François**
**42, Rue J. Georget**
**F-92140 Clamart (FR)**
Inventeur : **Sicsic, Sames**
**27, Rue Madeleine**
**F-92160 Antony (FR)**
Inventeur : **Le Bigot, Jean-François**
**3, Place Auguste Delaune**
**F-94800 Villejuif (FR)**

(74) Mandataire : **Ores, Irène et al**
**CABINET ORES 6, Avenue de Messine**
**F-75008 - Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 017 550 B1

Nouveau procédé de dosage des aminoglycosides et réactifs pour la mise en œuvre de ce procédé.

La présente invention est relative à un nouveau procédé de dosage des aminoglycosides, ainsi qu'à de nouveaux réactifs pour la réalisation de ce dosage.

Les aminoglycosides, ou oligosaccharides, constituent un important groupe d'antibiotiques basiques de structure chimique similaire et comportent généralement la désoxystreptamine (commune à tous les aminoglycosides à l'exception de la streptomycine, de la spectinomycine et de la kasugamycine) liée à d'autres sucres aminés variables selon l'aminoglycoside considéré. Ces sucres aminés sont la glucosamine, la méthyl-glucosamine, la kanosamine, la néobiosamine, la gentosamine, la garosamine, la purpurosamine, etc...

Les aminoglycosides qui ont un spectre d'action large, sont des médicaments toxiques : néphrotoxiques ou ototoxiques (ototoxicité à prédominance vestibulaire ou à prédominance cochléaire). Aussi est-il très important de suivre avec précision le taux de ces antibiotiques dans les fluides biologiques ; cela devient même indispensable lorsque l'infection à traiter est grave ou lorsqu'elle survient chez un malade insuffisant rénal. Le dosage précis des aminoglycosides dans les différents fluides biologiques et en particulier dans le sang, est en outre nécessaire en ce qu'il apporte la preuve objective qu'une concentration d'antibiotique supérieure à la concentration minimale inhibitrice (CMI), de la bactérie, est présente dans la circulation sanguine ; ce dosage permet, d'autre part, de vérifier que la teneur d'antibiotique est dans les limites acceptées de tolérance du produit, surtout lorsque le produit utilisé présente une marge relativement étroite (et c'est souvent le cas) entre les taux souhaités pour traiter les malades et les taux réputés limites de la toxicité. C'est la raison pour laquelle plusieurs méthodes de dosage des antibiotiques en général et des amino-glycosides en particulier, ont été mises au point : cependant, aucune méthode préconisée jusqu'ici ne satisfait aux cinq critères énumérés ci-après qui sont non seulement utiles mais nécessaires, voire indispensables :

— réaliser un dosage précis ; reproductible ; rapide ; simple ; peu onéreux.

C'est ainsi qu'ont été proposés :

— des dosages biologiques par diffusion en gélose [cf. en particulier les travaux de JUSTESEN et TAGE dans Acta. Pathol. microbiol. Scand. (1973) *241*, 111-114 ou de DOIVA et JACKSON dans J. Infect. Dis. (1975) *132* 399-406]. Ces dosages reposent sur la comparaison de l'activité bactériostatique de l'échantillon à doser vis-à-vis de témoins contenant des quantités connues d'antibiotique. Ils sont longs et difficilement reproductibles d'un laboratoire à un autre ;

— des dosages microbiologiques rapides [cf. en particulier les travaux de FENTON et alia dans « 9$^e$ Congrès International de Chimiothérapie » LONDRES juillet 1975 ou NOONE et alia dans Soc. Appli. Bacteriol. Tech. Ser. (1975) *8* 153-167]. Ces dosages plus utilisés, car plus rapides et qui reposent en général sur l'amplitude de la variation du pH du milieu dans lequel se trouvent un germe test et l'antibiotique à doser, ne sont pas très précis et ne peuvent pas être utilisés dans de nombreux cas où la précision est indispensable ;

— des dosages par chromatographie liquide haute performance (HPLC) décrits par exemple par ANHALT et JOHN dans Antimicrobiol. Agents and Chemother. (1977) *11* (4) 651-655. Cette chromatographie qui est couplée, en sortie de colonne, à un système de détection (par exemple un réfractomètre) constitue également un moyen de séparer et de doser les aminoglycosides. Ce procédé de dosage est onéreux et le matériel qu'il nécessite est complexe et coûteux ;

— des dosages radioimmunologiques [cf. en particulier les travaux de MAHON et alia dans Antimicrobiol. Agents and Chemother. (1973) *3* 585-589 ou de BROUGHTON et STRONG dans Chin. Chim. Acta (1976) *66* 125-129]. Ces dosages sont basés sur la compétition d'un antibiotique radio-actif et de l'antibiotique à doser pour des anticorps anti-antibiotiques. Une courbe-étalon préalablement établie permet un dosage rapide et précis. Malheureusement, cette méthode présente deux inconvénients majeurs : la préparation d'anticorps anti-antibiotiques et le comptage d'un élément radioactif ;

— des dosages radioenzymatiques [cf. en particulier les travaux de F. LE GOFFIC et J. F. ACAR dans Méthode de dosage de la gentamicine (1973) Monographie de la Gazette Médicale de France ou de BROUGHALL et REEWES dans J. Clin. Pathol. Microbiol. (1975) *28* 140-145]. Ces dosages consistent en la transformation quantitative de l'antibiotique à doser par une enzyme d'inactivation, en un produit dosable. Les enzymes d'inactivation utilisant des cofacteurs (acétyl CoA, ATP), la technique proposée pour doser le produit de la réaction consiste en l'utilisation de cofacteurs radioactifs. On obtient de cette manière un antibiotique modifié radioactif, dosable. Cette méthode de dosage qui est très précise, présente toutefois plusieurs inconvénients :

l'utilisation de composés radioactifs est toujours délicate et nécessite des précautions particulières ;

ces composés sont très coûteux et impliquent, pour leur dosage, un matériel trop lourd pour des laboratoires courants d'analyses médicales.

On a également préconisé (Technicon Instruments Corporation, FR-A-2 348 492) un procédé pour la détermination immunologique d'antibiotiques aminoglycosidiques caractérisé en ce qu'il consiste à former un mélange de l'échantillon, d'un composé à marquage fluorescent et d'un anticorps dirigé contre l'antibiotique à étudier et le composé et à mesurer la fluorescence du mélange ainsi formé pour déterminer la quantité d'antibiotique aminoglycosidique présente dans l'échantillon.

Ce procédé (qui n'est pas un procédé de dosage enzymatique) fait appel à une réaction immunologique (précédée d'ailleurs d'une réaction chimique), n'est pas très précis et surtout, est très délicat à réaliser.

La présente invention s'est en conséquence donnée pour but de pourvoir à une méthode de dosage des aminoglycosides qui soit rapide, peu onéreuse, simple d'emploi, ne nécessitant aucune précaution particulière, ne mettant pas en œuvre des composés radioactifs, particulièrement précise et reproductible.

La présente invention a pour objet un nouveau procédé de dosage des aminoglycosides, caractérisé en ce que l'on effectue la modification de l'aminoglycoside à doser, par des réactions enzymatiques spécifiques et contrôlées à l'aide de cofacteurs fluorescents et d'enzymes choisies parmi celles ayant pour substrat des aminoglycosides possédant le cycle « désoxystreptamine » et en ce que l'on effectue la lecture de l'intensité de la fluorescence du produit ainsi obtenu.

Le principe de ce dosage repose sur la modification de l'antibiotique par « branchement » d'un marqueur fluorescent sur la molécule. On obtient ainsi un antibiotique fluorescent facilement dosable. Cette modification par branchement du marqueur fluorescent peut être réalisée chimiquement : on dispose en effet de plusieurs réactifs fluorescents et notamment des amines. Mais comme les aminoglycosides sont des molécules polyfonctionnelles possédant plusieurs fonctions amines et plusieurs fonctions alcools de réactivités plus ou moins proches, il est donc très difficile de contrôler de telles modifications, de savoir combien de marqueurs ont réagi sur la molécule, et, partant, de pouvoir doser avec précision l'antibiotique marqué. Afin de pallier cet inconvénient, la Demanderesse a mis au point et a utilisé la sélectivité des réactions enzymatiques pour aboutir au dosage fluoroenzymatique conforme à la présente invention.

Il est connu en effet que les aminoglycosides peuvent être modifiés par des enzymes d'inactivation par acétylation, phosphorylation et nucléotidylation. Ces enzymes d'inactivation utilisent par exemple comme cofacteurs l'acétylcoenzyme A (AcCoA) et l'adénosine triphosphate (ATP). Ainsi la Demanderesse, en utilisant comme cofacteur un analogue fluorescent, a pu brancher de manière parfaitement sélective un marqueur et un seul sur une seule fonction prédéterminée de l'antibiotique.

Suivant un mode de réalisation avantageux du procédé objet de la présente invention, les cofacteurs utilisés sont des cofacteurs fluorescents du type éthénonucléotide, analogues des nucléotides triphosphates possédant le noyau adénine ou le noyau cytosine.

Le schéma réactionnel du dosage pourrait donc être représenté de la manière suivante :

$$\text{Aminoglycoside} + \varepsilon\text{NTP} \xrightarrow{\text{enzyme}} \varepsilon\text{NMP-Aminoglycoside} + \text{PPi (fluorescent dosable)}$$

(par exemple dans le sérum)

εNTP = éthénonucléotide-triphosphate,
εNMP = éthénonucléotide-monophosphate.

Suivant un autre mode de réalisation avantageux de l'objet de l'invention, les enzymes choisies pour modifier les aminoglycosides sont des nucléotidyltransférases.

Selon une modalité particulière de ce mode de réalisation, la nucléotidyltransférase choisie est une 4'aminoglycosidenucléotidyltransférase (ANT (4')) qui catalyse l'adénylation de la fonction alcool en 4' du cycle purpurosamine des aminoglycosides.

Cette enzyme peut être obtenue suivant — par exemple — la méthode décrite par F. LE GOFFIC et coll. dans Ann. Microbio. (Inst. Pasteur), *127*, 391-399.

Selon une autre modalité particulière de l'invention, la nucléotidyltransférase choisie est une 2''aminoglycosidenucléotidyltransférase (ANT (2'')) qui catalyse l'adénylation de l'hydroxyle en 2'' du cycle garosamine ou kanosamine des aminoglycosides.

Cette enzyme peut être obtenue par exemple suivant la technique décrite par BENVENISTE et DAVIES (1971) dans Febs. Letters, *14*, 293.

Ainsi donc, tout aminoglycoside contenant la déoxy-2-streptamine et possédant une fonction alcool en 4' ou en 2'', peut être adénylé par l'une ou l'autre de ces enzymes.

Un problème demeure toutefois encore : afin que ce dosage simple, rapide et précis puisse être utilisé à grande échelle dans tous les laboratoires d'analyse, il est indispensable de surmonter le handicap que constitue l'instabilité particulière des enzymes d'inactivation et notamment la thermolabilité des nucléotidyltransférases : leur activité ne peut être conservée qu'en les transportant et en les stockant à des basses températures, de l'ordre de − 20 °C.

Conformément à l'invention et afin de rendre la méthode d'un emploi facile et d'un prix de revient peu élevé, les enzymes d'inactivation des aminoglycosides sont stabilisées par immobilisation sur support soluble.

Suivant un mode de réalisation avantageux du procédé de dosage objet de la présente invention, les enzymes d'inactivation utilisées le sont sous forme d'extrait brut, après lyse des bactéries.

Des travaux récents (1977) ont en effet démontré que les enzymes d'inactivation non purifiées se trouvant dans un environnement stabilisant, supportent mieux l'immobilisation que les enzymes purifiées (cf. notamment les travaux de LE GOFFIC, SICSIC et VINCENT dans Biochimie *59* 927-932).

Conformément à l'invention, la stabilisation par immobilisation a été réalisée par réticulation avec le glutaraldéhyde sur un support soluble du type dextrane.

Il est en effet nécessaire, lors de l'immobilisation, de préserver absolument le site actif. Il y a une forte probabilité pour que le site de fixation des aminoglycosides soit constitué d'une cavité à caractère anionique, les substrats étant polyaminés. La Demanderesse a, en tenant compte de cette caractéristique, mis au point une immobilisation par participation des fonctions amines, l'immobilisation covalente utilisant les fonctions carboxyliques de ces enzymes étant susceptibles de les inactiver.

Le choix du support a été déterminé par sa solubilité afin que le dosage soit facilement réalisable, qu'il ait lieu dans une phase homogène, dans un simple tube à hémolyse, et qu'il soit possible de prélever plusieurs fois le même nombre d'unités enzymatiques en prélevant un même volume d'enzyme immobilisée.

Suivant un mode de réalisation avantageux du procédé conforme à la présente invention, l'immobilisation est effectuée en procédant tout d'abord à l'absorption de l'extrait enzymatique sur le support, en réticulant ensuite par le glutaraldéhyde à 25 % dans l'eau et en finissant par une opération de congélation et décongélation.

Suivant un mode de réalisation particulièrement avantageux du procédé conforme à l'invention, la solution contenant l'enzyme immobilisée est conservée sous forme lyophilisée.

Outre les dispositions qui précèdent, l'invention comprend encore d'autres dispositions, qui ressortiront de la description qui va suivre.

La présente invention vise particulièrement le procédé de dosage des aminoglycosides à l'aide des opérations fluoroenzymatiques conforme aux dispositions qui précèdent, les réactifs (enzymes, supports, analogues fluorescents) nécessaires à ce dosage, les moyens propres à la mise en œuvre de ce procédé, ainsi que les procédés d'ensemble dans lesquels sont inclus le procédé et les réactifs conformes à la présente invention.

L'invention pourra être mieux comprise à l'aide du complément de description qui va suivre, qui se réfère à des exemples de mise en œuvre du procédé conforme à la présente invention et notamment à la réalisation de la synthèse d'un cofacteur fluorescent, à la stabilisation des enzymes d'inactivation par immobilisation sur support soluble, à la réalisation de l'hémisynthèse d'antibiotiques modifiés fluorescents, au dosage par fluorescence des antibiotiques modifiés, et au protocole expérimental réalisant la transformation quantitative de l'antibiotique à doser dans un fluide biologique, en produit fluorescent et le dosage de ce produit par fluorescence.

Exemple 1 — Synthèse du cofacteur fluorescent

La préparation du cofacteur fluorescent (le 1N6 éthéno ATP représenté par la formule I ci-après) :

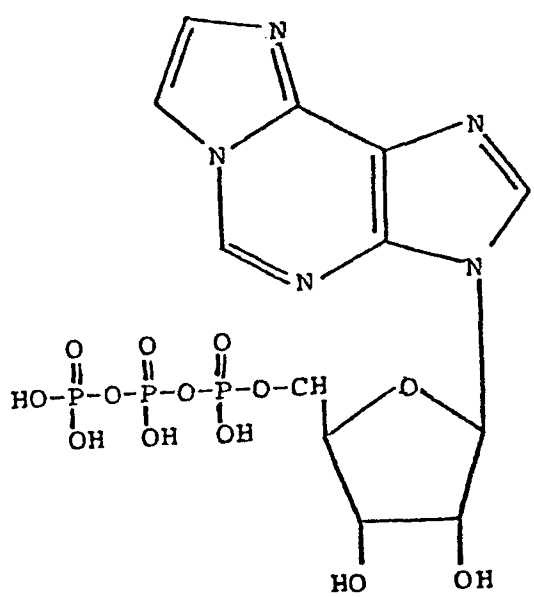

(I)

a été réalisée en s'inspirant de la méthode décrite par KOCHETOV dans « TETRAHEDRON LETTERS » (1971), *22*, 1993-1996 et par SECRIST et LEONARD dans J. Amer. Chem. Soc. (1972), *11*, 3499-3506.

En milieu légèrement acide, le chloroacétaldéhyde réagit sur l'adénine selon le schéma suivant :

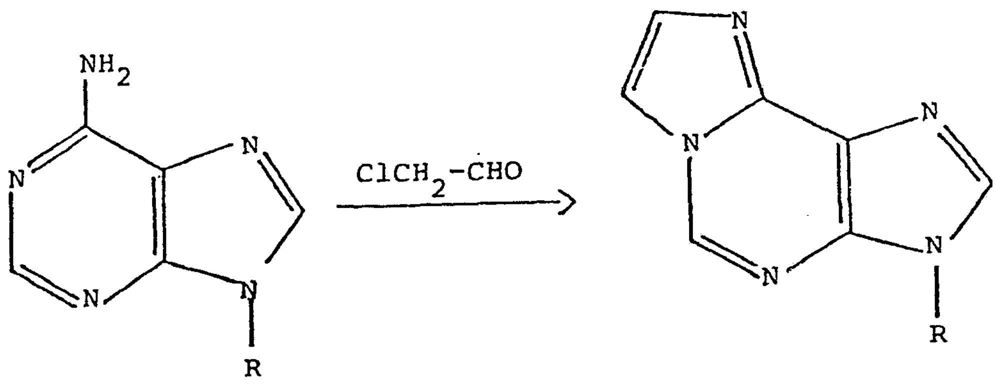


(II)

où R représente le résidu ribosyl-triphosphate.

La réaction est totale en 18 heures à 37 °C. La disparition de l'ATP est contrôlée en effectuant régulièrement des chromatographies sur couche mince. Quand la totalité de l'ATP a réagi, le chloroacétaldéhyde en excès est extrait. Le produit peut alors être purifié par simple chromatographie sur colonne de gel d'exclusion (dessalage). L'ATP utilisé est légèrement radioactif au $^{14}C$ sur le carbone en 8 de l'adénine. Cela permet par la suite de réaliser simultanément le dosage radioenzymatique et fluoroenzymatique à des fins de comparaison. On procédera par exemple ainsi :

30 ml d'une solution aqueuse de chloroacétaldéhyde 2M sont ajustés à pH = 4,5 par une solution de soude 1M. On ajoute alors 1,3 g d'ATP (Boehringer) et 50 μCi de $^{14}C$ ATP à 53 mCi/mmole (CEA Saclay). La température du mélange réactionnel est maintenue à 37 °C et le pH à 4,5.

L'avancement de la réaction est contrôlé par chromatographie sur couche mince (gel de silice) avec comme éluant un mélange 25 : 5 : 5 de méthanol d'ammoniaque à 20 % et d'eau (V/V/V). Au bout de 38 heures, tout l'ATP a disparu. Le chloroacétaldéhyde en excès est extrait par six fois 20 ml d'éther. Le produit est alors dessalé par chromatographie sur gel d'exclusion « SEPHADEX » G10 et lyophilisé. On obtient ainsi 2 mmoles d'εATP à 23,3 μCi/mmole.

Exemple 2 — Préparation de l'extrait enzymatique

Tampon A :
Tris HCl $10^{-2}$ M (pH = 7,5)
NaCl $3 \cdot 10^{-2}$ M

Tampon B :
Tris HCl $10^{-2}$ M (pH = 7,5)
$NH_4Cl$ $6 \cdot 10^{-2}$ M
$MgCl_2$ $10^{-2}$ M
β mercaptoéthanol $0,6 \cdot 10^{-3}$ M

Tampon C :
Tris HCl $10^{-1}$ M (pH = 7,5)
$NH_4Cl$ $6 \cdot 10^{-1}$ M
$MgCl_2$ $10^{-1}$ M
β mercaptoéthanol $0,6 \cdot 10^{-2}$ M

a) Culture des bactéries : A partir d'une colonie de Staphylococcus Aureus (Riv) ou Escherichia Coli R55 isolée sur boîte de Pétri, on ensemence dans un milieu complet Trypticase Soy Broth (30 g/l). Les bactéries sont récoltées en fin de phase exponentielle de croissance, par centrifugation (10 000 g/30 mm). Le culot d'un litre de culture est lavé trois fois par du tampon A.

b) Lyse des bactéries : Pour E. Coli R55, l'extraction se fait par désintégration des cellules. Après lavage, le culot est remis en suspension dans 20 ml de tampon B et soumis à une pression de 1 300 à 2 000 kg/cm². 5 mg de désoxyribonucléase (Boehringer) sont ajoutés et la préparation est laissée pendant 30 mn sous faible agitation dans un bain de glace. Elle est ensuite centrifugée à 40 000 g pendant 30 mn.

Pour Staphylococcus Aureus (Riv.), une fois le culot remis en suspension dans 20 ml de tampon B, les bactéries sont lysées par la lysostaphine (2 mg à 203 U/mg). Après action de la désoxyribonucléase, l'extrait est centrifugé à 40 000 g pendant 30 mn.

On obtient ainsi des extraits bruts de la 4'-amino-glycosidenucléotidyltransférase et de la 2"aminoglycoside-nucléotidyltransférase.

Exemple 3 — Evaluation de l'activité enzymatique des extraits obtenus

Dans un tube à hémolyse, on mélange 5 µl d'une solution de tobramycine à 2 µmole/ml, 5 µl de $^{14}C$ ATP à 10 µmole/ml (activité spécifique 1 µCi/µmole), 10 µl de tampon C ci-dessus et 20 µl d'extrait enzymatique (obtenu comme décrit ci-dessus).

Le mélange réactionnel, soit 40 µl, est incubé à 37 °C pendant 10 mn puis déposé sur un carré de papier de phosphocellulose (Whatman P 81). La réaction enzymatique est arrêtée par immersion du papier dans l'eau à 80 °C. On le lave ensuite abondamment à l'eau froide pour éliminer l'excès d'ATP. Après séchage, la radioactivité retenue sur le papier est déterminée dans un liquide scintillant (5 g de PPO, 100 mg de POPOP dans 1 litre de toluène) par un spectromètre Intertechnique SL 30.

Cette radioactivité représente la quantité d'antibiotique adénylé, fixé sur le papier par échange d'ions. Elle représente donc l'activité enzymatique de l'extrait.

$$\frac{\text{radioactivité retenue}}{\text{radioactivité totale}}\ 5 \cdot 10^{-2}/10/0{,}02\ (\text{en } \mu/\text{mole/mn/ml d'extrait})$$

Exemple 4 — Immobilisation des extraits enzymatiques sur support soluble

L'extrait enzymatique est lentement décongelé. Le support DEAE-dextrane ou dextrane T500 (Pharmacia) est dissous délicatement dans l'extrait (100 mg de support par ml d'extrait). A la solution ainsi obtenue, on ajoute le glutaraldéhyde à 25 % dans l'eau (Eastman Kodak) (1,4 µl de glutaraldéhyde/ml d'extrait) et on homogénéise la préparation qui est ensuite congelée pendant 2 heures. Après lente décongélation, l'activité enzymatique peut être déterminée comme indiqué à l'Exemple 3 ci-dessus.

Pour chaque mesure, un témoin sans antibiotique est réalisé. Cette précaution s'est révélée particulièrement utile quand le support utilisé pour l'immobilisation est le DEAE-dextrane. En effet, la nature cationique du support le rend capable de fixer de l'ATP $^{14}C$ et de se fixer lui-même sur le papier de phosphocellulose, faussant ainsi les résultats.

Les solutions d'enzymes immobilisées ainsi préparées sont très actives. Leur stabilité en fonction du temps est remarquable. Les résultats sont reportés dans le Tableau I ci-après :

TABLEAU I

| Support | Température de stockage | Forme physique de la préparation | Pourcentage d'activité Après 8 jours | Après 15 jours | Après 30 jours |
|---|---|---|---|---|---|
| DEAE-Dextrane | 25 °C | liquide | 60 | — | — |
| Dextrane T500 | 25 °C | liquide | 50 | 20 | — |
| DEAE-Dextrane | 4 °C | liquide | 75 | 60 | 50 |
| Dextrane T500 | 4 °C | liquide | 90 | 80 | 75 |
| Dextrane T500 ou DEAE-Dextrane | 4 °C | lyophilisée | 100 | 100 | 100 |

Les tests d'activité ont été effectués tous les jours d'abord, puis toutes les semaines durant plusieurs mois. Comme on le voit sur le Tableau I, le type d'immobilisation conforme à l'invention est remarquable : alors que les enzymes non immobilisées perdent la totalité de leur activité en 2 à 5 jours, certaines préparations obtenues conformément à l'invention gardent plus de la moitié de leur activité durant plusieurs mois.

Exemple 5 — Préparation de réactifs d'adénylation

Exemple de préparation de 4'εAMP-tobramycine et de 2" εAMP-tobramycine

(Voir formule, page 7)

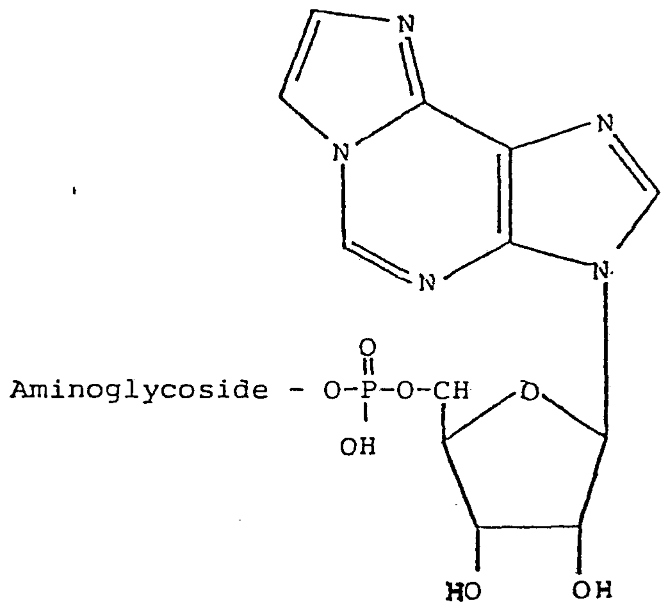

Ces hémisynthèses sont nécessaires pour deux raisons :

— pour étudier les propriétés de fluorescence de ce produit et sa stabilité dans différents milieux,

— pour mettre au point un protocole de détection et de détermination des concentrations du produit dans le sérum.

En effet, dans la plupart des cas, les composés fluorescents voient leur fluorescence perturbée dans le sérum. Celà est dû à des phénomènes de transfert d'énergie des composés fluorescents sur d'autres molécules sériques, protéiques en particulier.

Pour la réaction, on a utilisé un cofacteur légèrement radioactif ; on a pu, ainsi, suivre l'avancement de la réaction en mesurant la radioactivité retenue sur le papier de phosphocellulose avec ou sans rinçage. Au bout de 30 minutes, 80 % d'antibiotique sont transformés. En une heure, l'adénylation est totale.

On procède par exemple ainsi :

Dans 50 ml de tampon C (de l'Exemple 2) sont dissous 37,5 μmoles (18 mg) de tobramycine et 187,5 μmoles (100 mg) d'εATP $^{14}C$ (radioactivité spécifique 23,2 μCi/mmole). Le mélange est introduit dans une cellule d'ultra-filtration Amicon munie d'une membrane XM 100 A. 50 ml d'extrait enzymatique immobilisés et ultrafiltrés sont alors introduits. Après une heure, le rendement est quantitatif. Le produit est récupéré par ultrafiltration sous 2 bars. Après dessalage sur une colonne de gel d'exclusion G10, une chromatographie analytique sur couche mince (gel de silice) est pratiquée avec, comme éluant, un mélange 3 : 1 MeOH $NH_4OH$ 20 % (V/V). Les chromatogrammes sont révélés par l'iode et par du phosphomolybdate d'ammonium (réactif des sucres), la radioactivité est localisée au Scanner BERTHOLD, l'absorption UV et la fluorescence au photomètre VERNON.

3 taches sont observées :

— une tache radioactive et fluorescente, ne réagissant pas avec le phosphomolybdate d'ammonium, de Rf = 0,09. Il s'agit de l'εATP en excès ne migrant presque pas ;

— une tache non radioactive et ne présentant pas de fluorescence, réagissant avec le phosphomolybdate d'ammonium, de Rf = 0,3. Il s'agit d'un peu de tobramycine résiduelle ;

— une tache radioactive et fluorescente, réagissant avec le phosphomolybdate d'ammonium, de Rf = 0,5. C'est l'εAMP tobramycine.

Purification de l'εAMP tobramycine :

Le produit est repris dans 20 ml d'eau et l'on ajoute 5 g de résine CG 50 à 10,2 meq/g (« Amberlite » 100-200 mesh) préalablement équilibrée sous la forme $NH_4^+$ et lavée à l'eau distillée jusqu'à force ionique nulle. L'élution est pratiquée tout d'abord avec 50 ml d'eau. On récupère ainsi l'εATP en excès. Ensuite le produit est élué par un gradient linéaire d'ammoniaque (0 à 0,5 N). Une chromatographie analytique sur gel de silice réalisée dans le système décrit précédemment, montre la présence de deux taches : l'εAMP tobramycine et un peu de tobramycine.

Le produit est alors fixé par échange d'ions sur une résine échangeuse d'anions AG1 × 8 à 3,2 meq/g (Bio-Rad 200 à 400 mesh) préalablement équilibrée sous la forme formiate. Le pH étant fixé à 10,5, la résine est ajoutée jusqu'à disparition de la radioactivité dans la phase liquide. Elle est alors lavée avec 50 ml d'eau. Le produit est élué avec une solution d'acide formique 0,1 N. Il montre en chromatographie

analytique une tache unique de Rf = 0,5. 18 mg de 4′ εAMP tobramycine et 20 mg de 2″ εAMP tobramycine ont ainsi été obtenus.

Exemple 6 — Protocole de dosage

a) dans l'eau : les mesures de la fluorescence de l'εAMP tobramycine sont réalisées sur un spectrophotofluorimètre AMINCO SPF 125 et on trace les spectres d'absorption et d'émission de l'εAMP tobramycine dans l'eau à différents pH.

A l'excitation, quatre maxima sont observés : 285 nm, 290 nm, 303 nm, 322 nm, qui représentent l'absorption d'énergie par le produit, mais aussi les différentes bandes d'émission de la lampe.

A l'émission, le produit présente un large spectre de 350 nm à 520 nm dont le maximum est à 420 nm.

C'est à pH = 7 que le produit présente la plus grande fluorescence. A pH = 1, la fluorescence est bien plus faible et à pH basique le produit se dégrade et l'intensité de fluorescence décroît en fonction du temps. Les longueurs d'onde donnant la meilleure sensibilité sont 303 nm pour l'excitation et 420 nm pour l'émission. A ces longueurs d'onde, l'intensité de fluorescence est importante puisqu'elle permet de déterminer des concentrations en produit de $6 \cdot 10^{-5}$ μmole/ml ($5 \cdot 10^{-2}$ μg/ml).

b) dans le sérum : dans le sérum ou le plasma, les mesures réalisées montrent une très forte inhibition de la fluorescence, et ne permettent de déterminer que les grandes concentrations en produit. Il a donc fallu mettre au point un protocole levant cette inhibition.

La méthode choisie pour lever cette inhibition de fluorescence est l'extraction du produit à doser, par chromatographie échangeuse d'ions. En passant l'échantillon de sérum sur une petite colonne échangeuse de cations CG50, l'εAMP tobramycine s'accroche fortement à la résine.

En rinçant abondamment la colonne, la plupart des composés sériques, et en particulier les protéines, sont éliminés. Il ne reste plus ensuite qu'à éluer le produit avec un tampon concentré à pH = 7, directement dans la cellule de lecture du fluorimètre. L'εAMP tobramycine présente alors une bonne intensité de fluorescence et l'on peut aisément déterminer des concentrations de $1{,}2 \cdot 10^{-3}$ μmole/ml (1 μg/ml).

Lors de la mise en œuvre du dosage proprement dit, cette méthode présente l'avantage de permettre l'élimination de l'εATP en excès qui, ne s'accrochant pas à la résine, est lui aussi élué lors du rinçage.

On procède par exemple comme suit :

a) transformation enzymatique : il faut déterminer d'abord les conditions expérimentales pour que, lors du dosage, la transformation de l'antibiotique soit totale. Pour cela, les activités spécifiques des préparations enzymatiques sont tout d'abord mesurées comme indiqué à l'Exemple 3.

Elles sont d'environ $5 \cdot 10^{-2}$ U/ml (1 unité catalysant la transformation de 1 μmole/mn). Pour le dosage, on a à transformer de 1 μg/ml à 25 μg/ml d'antibiotique soit $2 \cdot 10^{-3}$ à $5 \cdot 10^{-2}$ μmoles pour des prises d'essai de 1 ml. Il faut donc $5 \cdot 10^{-3}$ U pour que la transformation de l'échantillon le plus concentré soit totale en 10 mn. En mettant 1 ml d'enzyme par échantillon, on se place en excès de 10.

Pour vérifier ces conditions, une gamme de $10^{-3}$ à 0,1 μmole/ml (0,5 à 50 μg/ml) de tobramycine a été adénylée. Les résultats sont les suivants :

TABLEAU II

| Tobramycine dans l'échantillon | | $^{14}C$ ATP | Radioactivité retenue après rinçage |
|---|---|---|---|
| en μg | en mole | en mole | en cpm |
| 50 | $10^{-7}$ | $5 \cdot 10^{-7}$ | 76 400 |
| 25 | $5 \cdot 10^{-8}$ | $5 \cdot 10^{-7}$ | 41 800 |
| 10 | $2 \cdot 10^{-8}$ | $5 \cdot 10^{-7}$ | 18 000 |
| 5 | $10^{-8}$ | $5 \cdot 10^{-7}$ | 9 200 |
| 1 | $2 \cdot 10^{-9}$ | $5 \cdot 10^{-7}$ | 2 150 |
| 0,5 | $10^{-9}$ | $5 \cdot 10^{-7}$ | 960 |

b) extraction de l'εAMP-tobramycine : dans 1 ml de sérum on introduit $6 \cdot 10^{-2}$ μmoles (50 μg) d'εAMP tobramycine. La résine CG50 équilibrée sous forme $NH_3^+$ est ajoutée jusqu'à disparition de la radioactivité dans la phase liquide (100 mg ≡ 1 meq.). Elle est ensuite rincée avec 50 ml d'eau distillée. 2 ml de tampon phosphate de potassium 1M pH = 7 servent à l'élution du produit. Cet éluat présente une intensité de fluorescence de 40 unités relatives. On élue donc une petite colonne de capacité 1 meq. avec une quantité de tampon représentant 2 meq. L'élution n'est donc pas totale. Malgré cela si les conditions d'extraction sont toujours identiques (même quantité de résine et même quantité de tampon éluant), l'intensité de fluorescence de l'éluat doit être proportionnelle à la quantité d'εAMP-tobramycine présente dans le sérum de départ. Cela a été vérifié comme représenté au Tableau III ci-dessous :

TABLEAU III

| Concentration d'εAMP tobramycine dans le sérum | | Intensité de fluorescence |
|---|---|---|
| en µg/ml | en µmole/ml | |
| 50 | $6 \cdot 10^{-2}$ | 85 |
| 20 | $2,4 \cdot 10^{-2}$ | 34 |
| 10 | $1,2 \cdot 10^{-2}$ | 17,5 |
| 4 | $5 \cdot 10^{-3}$ | 6 |
| 2 | $2,4 \cdot 10^{-3}$ | 3,2 |
| 1 | $1,2 \cdot 10^{-3}$ | 2 |
| 0 | 0 | 0,8 |

c) dosage de la tobramycine dans le sérum : comme pour bien des dosages il faut avant tout tracer une courbe-étalon. Pour cela dans des échantillons de sérum de 1 ml, sont introduites des quantités croissantes de tobramycine de $10^{-3}$ à 0,1 mole (0,5 à 50 µg). Dans chaque tube, 50 µl d'une solution d'εATP à 7,11 mg/ml (soit un excès de 5 pour l'échantillon le plus concentré en antibiotique) sont ajoutés.

$5 \cdot 10^{-2}$ unités enzymatiques sont alors ajoutées.

Après 10 mn d'incubation, chaque échantillon est déposé sur une petite colonne contenant 100 mg de résine CG50.$NH_3^+$. Après rinçage à l'eau distillée, chaque colonne est éluée avec 2 ml de tampon phosphate de potassium 1M pH = 7. Cet éluat est introduit dans la cellule de lecture du fluorimètre. La longueur d'onde d'excitation est réglée à 303 nm et la longueur d'onde d'émission à 420 nm.

On opère de la même façon quelle que soit l'aminoglycoside à doser. En se rapportant à la courbe-étalon, il est facile de déterminer la concentration en antibiotique, connaissant l'intensité de fluorescence.

Des expériences de dosage réalisées en aveugle ont ainsi permis de déterminer des concentrations de $4 \cdot 10^{-2}$ à $10^{-3}$ µmole/ml (30 à 1 µg/ml) avec une erreur inférieure à $10^{-3}$ µmole/ml. La courbe-étalon ainsi que les dosages réalisés en aveugle, montrent que la méthode est très précise et particulièrement reproductible.

**Revendications**

1. Procédé de dosage des aminoglycosides utilisant des composés à marquage fluorescent, caractérisé en ce que l'on effectue la modification de l'aminoglycoside à doser, par des réactions enzymatiques spécifiques et contrôlées à l'aide de cofacteurs fluorescents, du type éthénonucléotide analogues des nucléotides triphosphates possédant le noyau adénine ou le noyau cytosine, et de nucléotidyltransférases ayant pour substrat des aminoglycosides possédant le cycle « désoxystreptamine », et en ce que l'on effectue la lecture de l'intensité de la fluorescence du produit ainsi obtenu.

2. Procédé selon la revendication 1, caractérisé en ce que les nucléotidyltransférases mises en œuvre sont choisies parmi les 4' aminoglycosidénucléotidyltransférases (ANT(4')) qui catalysent l'adénylation de la fonction alcool en 4' du cycle purpurosamine des aminoglycosides.

3. Procédé selon la revendication 1, caractérisé en ce que les nucléotidyltransférases mises en œuvre sont choisies parmi les 2'' aminoglycosidenucléotidyltransférases (ANT(2'')) qui catalysent l'adénylation de l'hydroxyle en 2'' du cycle garosamine ou kanosamine des aminoglycosides.

4. Procédé selon l'une quelconque des revendications 1 à 3, caractérisé en ce que les enzymes d'inactivation des aminoglycosides sont stabilisées par immobilisation sur support soluble.

5. Procédé selon l'une quelconque des revendications 1 à 4, caractérisé en ce que les enzymes d'inactivation utilisées le sont sous forme d'extrait brut.

6. Procédé selon les revendications 1 et 4, caractérisé en ce que la stabilisation par immobilisation a été réalisée par réticulation avec le glutaraldéhyde sur un support soluble du type dextrane.

7. Procédé selon la revendication 6, caractérisé en ce que l'immobilisation est effectuée en procédant tout d'abord à l'absorption de l'extrait enzymatique sur le support, en réticulant ensuite par le glutaraldéhyde à 25 % environ dans l'eau, et en finissant par une opération de congélation et décongélation.

8. Procédé selon l'une quelconque des revendications 1 à 7, caractérisé en ce que la solution contenant l'enzyme immobilisée est conservée sous forme lyophilisée.

9. Produit utile pour le dosage des aminoglycosides selon l'une quelconque des revendications 1 à 8, caractérisé en ce qu'il est constitué des éthénonucléotide-monophosphates des aminoglycosides, εNMP-aminoglycosides.

**Claims**

1. Method of determining aminoglycosides using compounds with fluorescent marking, characterised in that the modification of the aminoglycoside to be determined is carried out, by specific enzymatic reactions and controlled by means of fluorescent cofactors, of the ethenonucleotide type, similar to triphosphate nucleotides possessing anadenine nucleus or cytosine nucleus, and nucleotidyltransferases having for substrate aminoglucosides having the « desoxystreptamine » ring and carrying out reading of the intensity of the fluorescence of the product thus-obtained.

2. Method according to claim 1, characterised in that the nucleotidyltransferases applied are selected from among 4'aminoglycosidenucleotidyltransferases (ANT(4')) which catalyse the adenylation of the alcohol function at the 4' position of the purpurosamine ring of the aminoglycosides.

3. Method according to claim 1, characterised in that the nucleotidyltransferases applied are selected from among the 2"aminoglycosidenucleotidyltransferases (ANT(2")) which catalyse the adenylation of the hydroxyl at the 2" position of the garosamine or kanosamine ring of the aminoglycosides.

4. Method according to any one of claims 1 to 3, characterised in that the inactivation enzymes of the aminoglycosides are stabilized by immobilization on a soluble support.

5. Method according to any one of claims 1 to 4, characterised in that the inactivation enzymes used are in the form of a crude extract.

6. Method according to claims 1 and 4, characterised in that the stabilization by immobilization has been carried out by cross-linking with glutaraldehyde on a soluble support of the dextran type.

7. Method according to claim 6, characterised in that the immobilization is carried out by proceding first with the absorption of the enzymatic extract on the support, then by cross-linking with approximately 25 % glutaraldehyde in water, and by finishing by a freezing and thawing operation.

8. Method according to any one of claims 1 to 7, characterised in that the solution containing the immobilized enzyme is preserved in freeze-dried form.

9. Product of the ethenonucleotide type useful for the determination of amino-glycosides according to any one of claims 1 to 8, characterised in that it is constituted from ethenonucleotide-monophosphates of aminoglycosides, εNMP-aminoglycosides.

**Ansprüche**

1. Verfahren zur Bestimmung von Aminoglycosiden unter Verwendung von Verbindungen mit fluoreszierender Markierung, dadurch gekennzeichnet, daß man das zu bestimmende Aminoglycosid durch spezifische und gesteuerte enzymatische Reaktionen mit Hilfe von fluoreszierenden Cofaktoren vom Äthenonucleotid-Typ, Analogen von Nucleotid-Triphosphaten, die den Adeninkern oder den Cytosinkern aufweisen, und Nucleotidyltransferasen, die als Substrat Aminoglycoside mit dem « Desoxystreptamin »-Ring haben, modifiziert, und daß man die Intensität der Fluoreszenz des so erhaltenen Produkts abliest.

2. Verfahren nach Anspruch 1, dadurch gekennzeichnet, daß die eingesetzten Nucleotidyltransferasen ausgewählt werden unter den 4'-Aminoglycosidnucleotidyl-transferasen (ANT(4')), die die Adenylierung der Alkoholfunktion in 4'-Stellung des Purpurosaminringes der Aminoglycoside katalysieren.

3. Verfahren nach Anspruch 1, dadurch gekennzeichnet, daß die eingesetzten Nucleotidyltransferasen ausgewählt werden unter den 2"-Aminoglycosidnucleotidyltransferasen (ANT(2")), die die Adenylierung des Hydroxyls in 2" des Garosamin- oder Kanosamin-Ringes der Aminoglycoside katalysieren.

4. Verfahren nach einem der Ansprüche 1 bis 3, dadurch gekennzeichnet, daß die Inaktivierungs-Enzyme der Aminoglycoside stabilisiert werden durch Immobilisieren an einem löslichen Träger.

5. Verfahren nach einem der Ansprüche 1 bis 4, dadurch gekennzeichnet, daß die verwendeten Inaktivierungs-Enzyme in Form eines Rohextrakts verwendet werden.

6. Verfahren nach einem der Ansprüche 1 bis 4, dadurch gekennzeichnet, daß die Stabilisierung durch Immobilisieren durchgeführt wurde durch Vernetzen mit Glutaraldehyd auf einem löslichen Träger vom Dextrantyp.

7. Verfahren nach Anspruch 6, dadurch gekennzeichnet, daß die Immobilisierung dadurch bewirkt wird, daß man zunächst den enzymatischen Extrakt an dem Träger absorbiert, anschließend mit Glutaraldehyd von etwa 25 % in Wasser vernetzt und abschließend einen Ausfrier- und Auftau-Arbeitsgang durchführt.

8. Verfahren nach einem der Ansprüche 1 bis 7, dadurch gekennzeichnet, daß die das immobilisierte Enzym enthaltende Lösung in lyophilisierter Form konserviert wird.

9. Produkt, geeignet zur Bestimmung von Aminoglycosiden nach einem der Ansprüche 1 bis 8, dadurch gekennzeichnet, daß es aus Äthenonucleotid-Monophosphaten von Aminoglycosiden, εNMP-Aminoglycosiden, besteht.